# EUROPEAN PATENT APPLICATION

(11) **EP 1 953 922 A1**
(43) Date of publication of application: **06.08.2008**
(21) Application number: 07023196.4
(22) Date of filing: 30.11.2007
(51) Int. Cl.: H04B 1/04, A61N 1/372

(54) **Tansceiver for implantable medical devices**

(30) Priority: 30.01.2007 US 668492
(71) Applicant: BIOTRONIK CRM Patent AG, 6341 Baar (CH)
(72) Inventor: Sutton, Brian P., West Linn, OR 97068 (US)
(74) Representative: Lindner-Vogt, Karin L.

(57) **Abstract**

The invention relates to an implantable medical device having a telemetry circuit for bidirectional data communication between the implantable medical device and an external device, wherein said telemetry circuit comprises a transceiver unit that is electronically switchable between a fully-synthesized frequency agile transceiver configuration and a low power single channel configuration, wherein the transceiver unit consumes less power when operated in its low power single channel configuration than in its fully-synthesized frequency agile configuration.

## Description

The invention refers to implantable medical devices (IMDs) providing a transceiver for data communication with an external device for treatment of atrial fibrillation. The invention relates in particular to implantable cardiac pacemakers or an implantable cardioverter/defibrillators (ICDs).

Telemedicine-enabled pacemakers and ICDs that use radio frequency (RF) telemetry play a vital role in providing timely reporting of a patient's medical condition to a physician. By regular and frequent reporting of a patient's cardiac condition (and implant status) to his physician, the treatment therapy can be adjusted, as necessary, for best quality of life. However, the telemedicine features remain of secondary importance when compared to the primary function of the implant - regulating a patient's heartbeat and/or providing a life-saving shock to treat ventricular fibrillation. As such, careful consideration must be given to the design of the RF telemetry circuitry to ensure it has minimal impact on the power consumption and longevity of the pacemaker or ICD battery.

All electronic circuitry in an implant consumes power, and because of the finite energy available from the battery, considerable effort is expended to ensure the power consumption is minimized. Battery longevity is a critical issue since the patient must undergo a surgical procedure to replace an implant when its battery nears its depleted state. Since the telemedicine feature is of secondary importance to the therapeutic function of the implant, the power consumed by its circuitry must be minimal. The RF telemetry should only consume as much battery power as is necessary to establish a reliable communications link for the task at hand. Any additional power consumed serves no useful purpose to the patient, and only hastens the time when the patient must be subjected to another surgical procedure.

The nature of implant RF telemetry sessions differ depending upon the use-case. In some situations, the implant may be required to transmit brief status messages to a home monitoring center on a daily basis. In other cases, the implant may need to transmit lengthy IEGM data during a patient's semi-annual exam. Whatever the use-case, the battery power consumed by the RF telemetry function should be optimized for the application.

It is an object of the invention to provide an implantable medical device that provides an improved transceiver for data communication.

According to the present invention the object of the invention is achieved by an implantable medical device featuring a telemetry circuit for bidirectional data communication between the implantable medical device and an external device, wherein the telemetry circuit comprises a transceiver unit that is electronically switchable between a fully-synthesized frequency agile configuration and a low power single channel configuration, wherein the transceiver unit consumes less power when operated in its low power single channel configuration than in its fully-synthesized frequency agile configuration.

Preferably, the low power single channel configuration of the transceiver unit forms either a single channel frequency modulated (FM) frequency shift keying (FSK) transmitter or a single channel on-off-keying (OOK) transmitter.

For any configuration, it is preferred that the transceiver unit comprises a frequency synthesizer comprising a voltage controlled oscillator (VCO), wherein the frequency synthesizer is switchable between
- an open-loop configuration wherein the voltage controlled oscillator is operated in an open-loop mode for forming the low-power single channel configuration of the transceiver and
- a closed-loop configuration wherein the voltage controlled oscillator is part of a phase locked loop (PLL) of the frequency synthesizer for forming the fully-synthesized frequency agile configuration of the transceiver.

Preferably, a telemetry circuit control unit and a switch is provided with the transceiver unit. The switch is connected to the telemetry circuit control unit and to the voltage controlled oscillator and is adapted to be set by the telemetry circuit control unit so as to allow switching of the frequency synthesizer between its open-loop configuration and its closed-loop configuration upon a control signal of the telemetry circuit control unit.

With respect to the low power single channel configuration for frequency shift keying of data to be transmitted, it is preferred that the telemetry circuit control unit comprises a data input and that the voltage controlled oscillator comprises a signal output and frequency control input that is connected to the telemetry circuit control unit so as to enable a frequency modulation of a signal generated by the voltage controlled oscillator according to data applied to the telemetry circuit control unit's data input.

Alternatively and also with respect to the low power single channel configuration but for on-off keying of data to be transmitted, it is preferred that the telemetry circuit control unit comprises a data input and that the transceiver unit comprises an amplifier having an input connected to the voltage controlled oscillator for receiving fixed frequency carrier signal and a further input connected to the telemetry circuit control unit for controlling the amplifier gain or switching the amplifier on and off in order to on-off key the fixed frequency signal according to data applied to the telemetry circuit control unit's data input.

It is to be appreciated that features of preferred embodiments of the invention may be combined in any useful manner thus arriving a further preferred embodiments of the invention not explicitly mentioned in this disclosure.

The above and other aspects, features and advantages of the present invention will be more apparent from the following more particular description thereof, presented in conjunction with the following drawings wherein:
- FIG.: 1 shows a dual chamber pacemaker/ atrial defibrillator/cardioverter connected to leads placed in a heart.
- FIG. 2: is a block diagram of the device of figure 1.
- FIG. 3: is a simplified circuit diagram of a telemetry circuit comprising a transceiver unit according to the invention.
- FIG. 4: is a simplified circuit diagram of a fully-synthesized frequency agile transceiver.
- FIG. 5: is a simplified circuit diagram of a low power single channel transmitter.

The following description is of the best mode presently contemplated for carrying out the invention. This description is not to be taken in a limiting sense, but is made merely for the purpose of describing the general principles of the invention. The scope of the invention should be determined with reference to the claims.

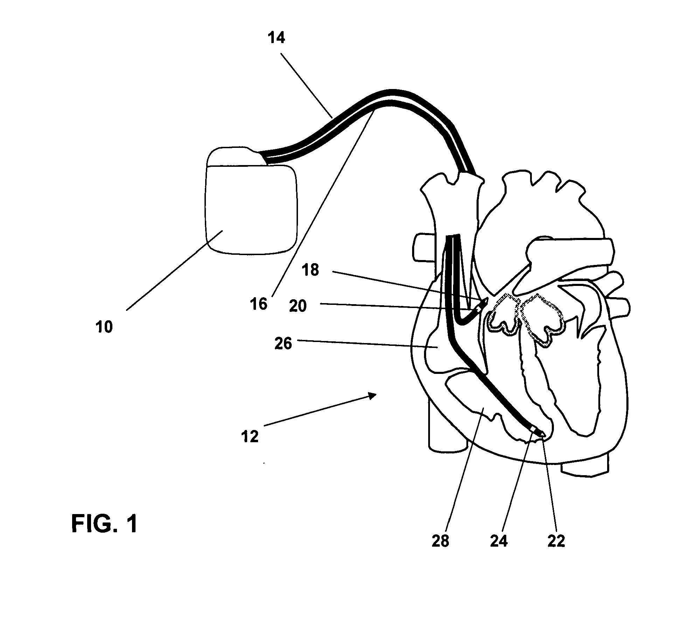

In FIG. 1 a dual chamber pacemaker 10 as heart stimulator is connected to pacing/sensing leads placed in a heart 12 is illustrated. The pacemaker 10 is electrically coupled to heart 12 by way of leads 14 and 16. Lead 14 has a pair of right atrial electrodes 18 and 20 that are in contact with the right atria 26 of the heart 12. Lead 16 has a pair of electrodes 22 and 24 that are in contact with the right ventricle 28 of heart 12. Electrodes 18 and 22 are tip-electrodes at the very distal end of leads 14 and 16, respectively. Electrode 18 is a right atrial tip electrode RA-Tip and electrode 22 is a right ventricular tip electrode RV-Tip. Electrodes 20 and 24 are ring electrodes in close proximity but electrically isolated from the respective tip electrodes 18 and 22. Electrode 20 forms a right atrial ring electrode RARing and electrode 24 forms a right ventricular ring electrode RV-Ring.

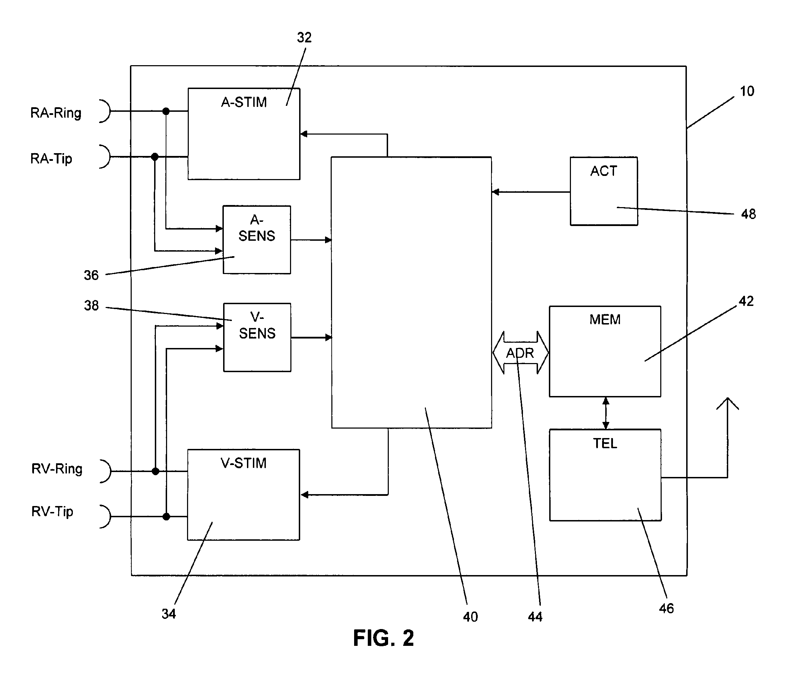

Referring to FIG. 2 a simplified block diagram of a dual chamber pacemaker 10 is illustrated. During operation of the pacemaker leads 14 and 16 are connected to respective output/input terminals of pacemaker 10 as indicated in Fig. 1 and carry stimulating pulses to the tip electrodes 18 and 22 from an atrial stimulation pulse generator A-STIM 32 and a ventricular pulse generator V-STIM 34, respectively. Further, electrical signals from the atrium are carried from the electrode pair 18 and 20, through the lead 14, to the input terminal of an atrial channel sensing stage A-SENS 36; and electrical signals from the ventricles are carried from the electrode pair 22 and 24, through the lead 16, to the input terminal of a ventricular sensing stage V-SENS 38.

Controlling the dual chamber pacer 10 is a control unit CTRL 40 that is connected to sensing stages A-SENS 36 and V-SENS 38 and to stimulation pulse generators A-STIM 32 and V-STIM 34. Control unit CTRL 40 receives the output signals from the atrial sensing stage A-SENS 36 and from the ventricular sensing stage V-SENS 38. The output signals of sensing stages A-SENS 36 and V-SENS 38 are generated each time that a P-wave representing an intrinsic atrial event or an R-wave representing an intrinsic ventricular event, respectively, is sensed within the heart 12. An As-signal is generated, when the atrial sensing stage A-SENS 36 detects a P-wave and a Vs-signal is generated, when the ventricular sensing stage V-SENS 38 detects an R-wave.

Control unit CTRL 40 also generates trigger signals that are sent to the atrial stimulation pulse generator A-STIM 32 and the ventricular stimulation pulse generator V-STIM 34, respectively. Control unit CTRL 40 comprises circuitry for timing ventricular and/or atrial stimulation pulses according to an adequate stimulation rate that can be adapted to a patient's hemodynamic need as pointed out below.

Still referring to FIG. 2, the pacer 10 includes a memory circuit MEM 42 that is coupled to the control unit CTRL 40 over a suitable data/address bus ADR 44. This memory circuit MEM 42 allows certain control parameters, used by the control unit CTRL 40 in controlling the operation of the pacemaker 10, to be programmably stored and modified, as required, in order to customize the pacemaker's operation to suit the needs of a particular patient. Such data includes basic timing intervals used during operation of the pacemaker 10 for triggering of ventricular or atrial stimulation pulses.

Further, data sensed during the operation of the pacemaker may be stored in the memory MEM 42 for later retrieval and analysis.

A telemetry circuit TEL 46 is further included in the pacemaker 10. This telemetry circuit TEL 46 is connected to the control unit CTRL 40 by way of a suitable command/data bus. Telemetry circuit TEL 46 allows for wireless data exchange between the pacemaker 10 and some remote programming or analyzing device which can be part of a centralized service center serving multiple pacemakers.

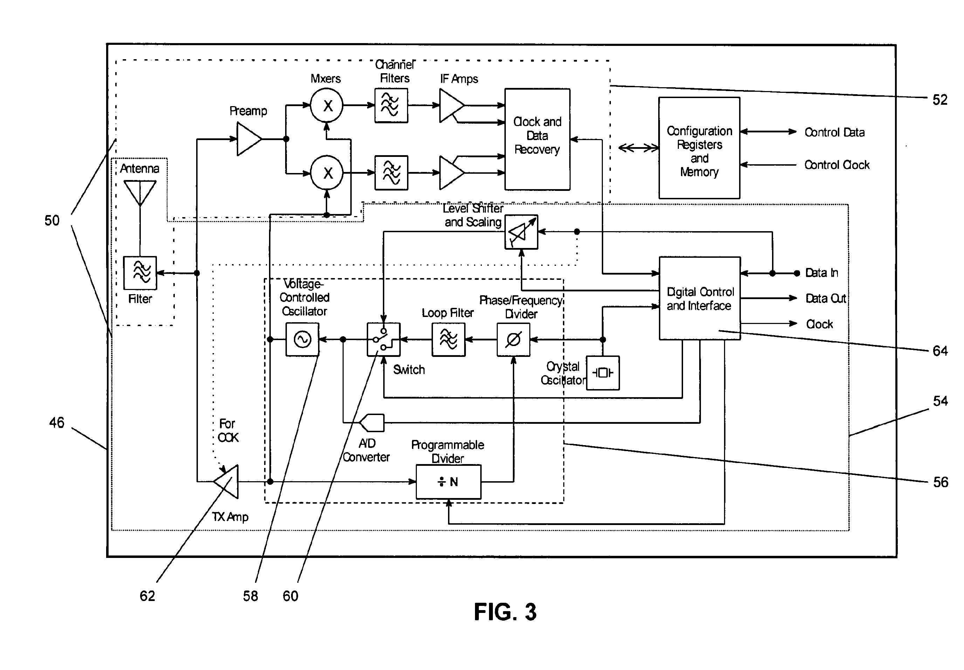

Turning now to figure 3, telemetry circuit TEL 46 comprises a transceiver unit 50 that is electronically switchable between a fully-synthesized frequency agile configuration and a low power single channel configuration, wherein the transceiver unit consumes less power when operated in its low power single channel configuration than in its fully-synthesized frequency agile configuration.

The transceiver unit 50 comprises a receiver 52 and a transmitter 54. The transmitter 54 and thus the transceiver 50 unit comprises a frequency synthesizer 56 that comprises a voltage controlled oscillator (VCO) 58 and a switch 60 that is connected to an input of the voltage controlled oscillator 58.

By means of the switch 60, the frequency synthesizer 56 is switchable between an open-loop configuration wherein the voltage controlled oscillator 58 is operated in an open-loop mode for forming said low-power single channel configuration of the transceiver 50 and a closed-loop configuration wherein the voltage controlled oscillator is part of a phase locked loop (PLL) of the frequency synthesizer 56 for forming said fully-synthesized frequency agile configuration of the transceiver 50.

The phase locked loop comprises the switch 60 and the voltage controlled oscillator 58, that is connected to a programmable divider, that in turn is connected to a phase/frequency divider, that in turn is connected to a loop filter that is connected to the VCO when the switch is set to its closed-loop setting.

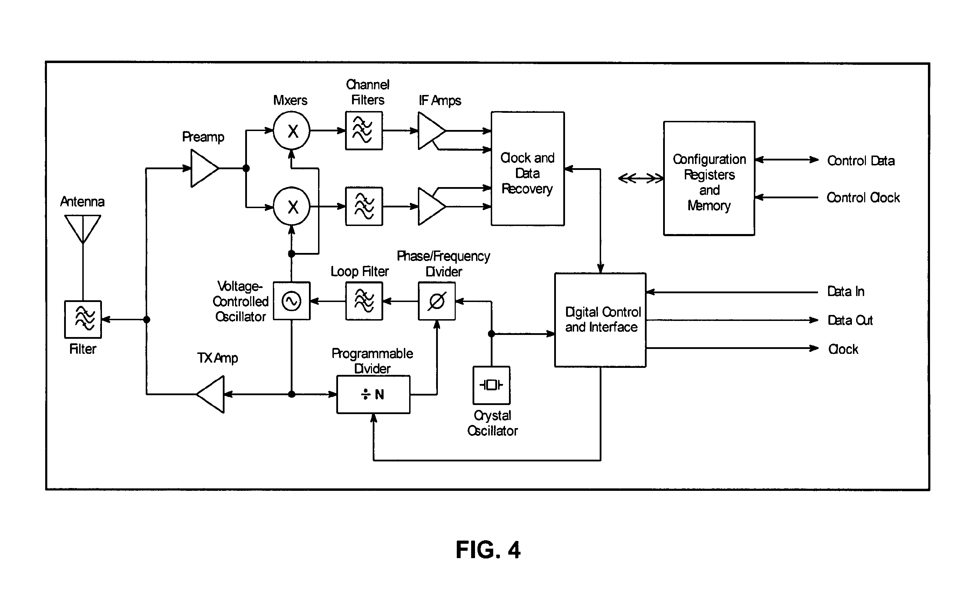

Further details of the fully-synthesized frequency agile configuration of the transceiver are disclosed with respect to figure 4.

If the switch 60 is set to form the open-loop configuration of the frequency synthesizer, the voltage controlled oscillator 58 is either set to generate a fixed frequency output signal for On-Off-Keying (OOK) or the voltage controlled oscillator is set to generate a frequency modulated (FM) output signal for Frequency-Shift-Keying (FSK). On-Off-Keying is performed by modulating a transmitter amplifier gain of a transmitter amplifier 62 that is connected to an output terminal of the voltage controlled oscillator 58. Modulating the amplifier's gain is most easily done by simply switching the amplifier 62 on and off. Alternatively, the gain of the amplifier 62 in its literal sense is controlled resulting in an amplitude modulated (AM) signal.

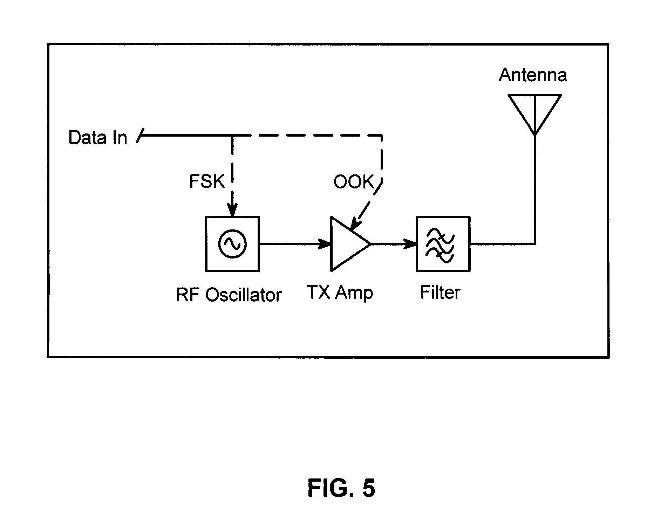

The preferred low power single channel configuration of the transceiver 50 is either a single channel frequency modulated (FM) frequency shift keying (FSK) transceiver or a single channel on-off-keying (OOK) transceiver as is further illustrated with respect to figure 5.

For controlling the switch 60 and either the transmitter amplifier's 62 gain or the voltage controlled oscillator's 58 frequency (either directly or via the PLL) according the data to be transmitted, a telemetry circuit control unit 64 is provided that is connected to the switch 60 so as to allow switching of the frequency synthesizer 62 between its open-loop configuration and its closed-loop configuration upon a control signal of said telemetry circuit control unit 64.

In the fully-synthesized frequency agile configuration of transceiver 50, both, the receiver 52 and the transmitter 54 are activated. Switch 60 would be set to the alternative setting not depicted in figure 3 thus closing the phase locked loop.

In the low power single channel configuration the receiver 52 is switched off. Switch 60 is set as depicted in figure 3. Thus the phase locked loop is deactivated and either the frequency of voltage controlled oscillator 58 or the transmitter amplifier are directly controlled by telemetry circuit control unit 64.

The telemetry circuit control unit 64 comprises a data input terminal and a control output terminal and is adapted to generate a control output signal for controlling either the voltage controlled oscillator's 58 frequency or for switching the transmitter amplifier 62 on and off according to data fed to telemetry circuit control unit's 64 data input terminal. Thus, either data to be transmitted can be frequency shift keyed or on-off-keyed.

Since the telemedicine requirements of medical implants can vary greatly - from sending short bursts of data very frequently, to sending long strings of data very infrequently, the following transceiver architecture is proposed to capitalize on the advantages of both simple single-channel RF transmitters and multi-channel, listen-before-transmit (LBT) compliant, frequency agile transceivers. The proposed architecture, as shown in Figure 3 employs a fully-synthesized frequency agile transceiver, as well as a means to electronically reconfigure the architecture to transform it into a simple, single-channel, ultra low-power transmitter.

When it is necessary to transmit large amounts of data (e.g. IEGM use-case), the architecture is configured to operate as a fully-synthesized frequency agile transceiver. The transceiver can select an unused channel to minimize interference, and can employ handshaking protocols to identify and eliminate transmission errors. All this capability, however, comes at the expense of higher power consumption and reduced battery life. In this operating mode, the phase locked loop (PLL) loop filter is connected by a switch to the tuning port of the voltage-controlled oscillator. The PLL behaves in its usual manner to phase-lock the VCO to the reference crystal oscillator. The A/D converter and Level Shifter and Scaling sections of the transceiver are not used, and are powered off to reduce power consumption.

At times when the implant only needs to send a small amount of patient data, and its operational status, (e.g. daily home monitoring use-case), the transceiver architecture is converted to an ultra low-power single-channel transmitter. The majority of the stages in the transceiver are switched off to conserve battery power. The only circuitry that is enabled and powered is the voltage-controlled oscillator in the frequency synthesizer, the level shifter and scaling circuitry, and the RF amplifier. Operation is now identical to the simple, ultra low-power, single channel transmitter. The cumulative power savings in this mode of operation are significant, and very important for battery longevity, since such operation may occur frequently (on a daily basis).

To implement the dual-mode RF transceiver, the frequency synthesizer has been modified to allow the voltage-controlled oscillator to be operated in "open-loop" mode. The included switch allows the transmit data to directly modulate the carrier frequency (for FM modulation) via a level shifter and scaling circuit. This circuit is used to establish the operating frequency and FM peak deviation. Alternatively, if OOK modulation is desired, the transmit data would modulate the power to the transceiver unit's amplifier.

An analog-to-digital converter (A/D) is used to measure the voltage-controlled oscillator's (VCO) tuning voltage when the frequency synthesizer is phase-locked on the desired channel. A digital-to-analog converter in the Digital Control and Interface section then applies this voltage to the VCO when the transmitter is used in open-loop mode, and PLL circuitry is powered off. The value of the digital-to-analog converter's setting is established at the factory during manufacturing test, and is stored in a non-volatile memory. Alternatively, the digital-to-analog setting can be updated periodically just prior to transmission by momentarily phase-locking the VCO to the reference crystal oscillator.

To further illustrate the structure and operation of the transceiver unit in its closed-loop configuration, in figure 4 a fully-synthesized frequency agile transceiver is depicted. Figure 4 shows the architecture of the fully-synthesized frequency agile transceiver. In its receive mode, typical current consumption is in the order of 10 mA, while the current consumption in transmit mode can be 15 mA or more.

In its low power single channel configuration the receiver part and the PLL circuitry of the transceiver unit are switched off. Figure 5 shows the architecture of the RF components that remain active in the low power single channel configuration of the transceiver unit and that form a single-channel, ultra low-power medical implant transmitter consuming typically less than 3 mA with a supply of 3 volts.

Although an exemplary embodiment of the present invention has been shown and described, it should be apparent to those of ordinary skill that a number of changes and modifications to the invention may be made without departing from the spirit and scope of the invention. In particular, it is possible to implement the features of the claimed transceiver unit into state of the art implantable medical devices such as implantable pacemakers or implantable cardioverter/defibrillator. This invention can readily be adapted to such devices by following the present teachings. All such changes, modifications and alterations should therefore be recognized as falling within the scope of the present invention.

## Claims

1. An implantable medical device having a telemetry circuit for bidirectional data communication between the implantable medical device and an external device, wherein said telemetry circuit comprises a transceiver unit that is electronically switchable between a fully-synthesized frequency agile transceiver configuration and a low power single channel configuration, wherein the transceiver unit consumes less power when operated in its low power single channel configuration than in its fully-synthesized frequency agile configuration.

2. The implantable medical device according to claim 1, wherein the low power single channel configuration of the transceiver unit forms a single channel frequency modulated (FM) frequency shift keying (FSK) transmitter.

3. The implantable medical device according to claim 1, wherein the low power single channel configuration of the transceiver unit forms a single channel on-off-keying (OOK) transmitter.

4. The implantable medical device according to claim 1, wherein the transceiver unit comprises a frequency synthesizer comprising a voltage controlled oscillator (VCO), wherein the frequency synthesizer is switchable between
an open-loop configuration wherein the voltage controlled oscillator is operated in an open-loop mode for forming said low-power single transmitter configuration of the transceiver unit and
a closed-loop configuration wherein the voltage controlled oscillator is part of a phase locked loop (PLL) of the frequency synthesizer for forming said fully-synthesized frequency agile configuration of the transceiver unit.

5. The implantable medical device according to claim 4, wherein the telemetry circuit comprises a telemetry circuit control unit and wherein the transceiver unit comprises a switch that is connected to said telemetry circuit control unit and to said voltage controlled oscillator and is adapted to be switched by said telemetry circuit control unit so as to allow switching of said frequency synthesizer between its open-loop transmitter configuration and its closed-loop configuration upon a control signal of said telemetry circuit control unit.

6. The implantable medical device according to claim 2 and 5, wherein said telemetry circuit control unit comprises a data input and wherein said voltage controlled oscillator comprises a signal output and frequency control input that is connected to said telemetry circuit control unit so as to enable a frequency modulation of a signal generated by said voltage controlled oscillator according to data applied to said telemetry circuit control unit's data input.

7. The implantable medical device according to claim 3 and 5, wherein said telemetry circuit control unit comprises a data input and wherein the transceiver unit comprises an amplifier having an input connected to the voltage controlled oscillator for receiving fixed frequency carrier signal and a further input connected to said telemetry circuit control unit for controlling the amplifier gain or switching the amplifier on and off in order to on-off key the fixed frequency signal according to data applied to said telemetry circuit control unit's data input.

8. The implantable medical device according to claim 1, wherein the transceiver unit comprises a receiver unit that is switched off if when the transceiver unit is operated in its low power single channel configuration.

9. The implantable medical device according to claim 1, wherein the implantable medical device is a cardiac pacemaker.

10. The implantable medical device according to claim 1, wherein the implantable medical device is a cardioverter/defibrillator.
